# EUROPEAN PATENT SPECIFICATION

(11) **EP 3 857 204 B1**
(45) Date of publication and mention of the grant of the patent: **19.04.2023**
(21) Application number: 19779741.8
(22) Date of filing: 17.09.2019
(51) Int. Cl.: G01N 21/31, G01N 21/64, G01N 33/14, G01N 33/52, G01N 21/17, G01N 21/75

(54) **A METHOD FOR IDENTIFYING THE PROPERTIES OF A SAMPLE, IN PARTICULAR A LIQUID SAMPLE**
VERFAHREN ZUR IDENTIFIZIERUNG DER EIGENSCHAFTEN EINER PROBE, INSBESONDERE EINER FLÜSSIGEN PROBE
PROCEDE D'IDENTIFICATION DES PROPRIETES D'UN ECHANTILLON, EN PARTICULIER D'UN ECHANTILLON LIQUIDE

(30) Priority: 27.09.2018 CZ 20180510
(43) Date of publication of application: 04.08.2021
(73) Proprietor: Mendelova Univerzita v Brne, 61300 Brno (CZ)
(72) Inventor: NEJDL, Lukás, 615 00 Brno (CZ); ADAM, Vojtech, 602 00 Brno (CZ); VACULOVICOVÁ, Markéta, 615 00 Brno (CZ)
(74) Representative: Plicka, Josef
(86) International application number: PCT/CZ2019/000046
(87) International publication number: WO 2020/064031

(56) References cited:
- US-A1- 2007 141 726
- US-A1- 2013 165 330
- US-B1- 6 313 274

## Description

### Technical Field

The invention relates to a method for identifying the properties of a sample, in particular a liquid sample, including liquefied samples, including biological samples, thus being included in the field of analytical chemistry. For identifying, the method uses natural spectral properties of substances, i.e. the absorption and emission of radiation, which varies depending on UV irradiation. Spectral analysis of these changes and chemical reactions yields valuable information on the physical, chemical or biological properties of the sample in a short time, without the need for any previously complicated preparation of the sample under investigation.

### Background Art

The diverse composition of pharmacological products, especially drugs, drug mixtures, i.e. addictive and psychotropic substances, foods, beverages, and biological or clinical specimens, such as blood plasma or urine, is a fundamental problem that makes it difficult to analyze them within tens of minutes.

In most cases, an extraction, isolation and/or separation process must be performed prior to an analysis. The sample thus treated is subjected to an instrumental analysis performed by a highly qualified person using complicated and costly instrumentation. The whole process, though precise, is time-consuming and costly.

A rapid drug determination may be crucial in rescuing a patient overdosed with an unknown drug during the transportation to a hospital. It is particularly important to detect a faulty or counterfeit drug batch before it is distributed. The ability to recognize important properties of the test substance within minutes appears to be crucial for the industry or food industry. In particular, this may involve checking the origin of cask wines or the quality control of the technological processing of fruit juices and vegetable extracts or beverages containing these juices and extracts.

In recent years, efforts have been exerted, particularly in the medical field, to develop methods which employ typical shapes or patterns of spectra, sometimes referred to as so-called "fingerprints". These methods are based in particular on spectral analysis, for example Raman spectroscopy, Fourier transform infrared spectroscopy and clinical specimen mass spectrometry, wherein the shapes of the recorded spectra are evaluated. Although these methods do not allow precise determination of the quantitative and qualitative representation of individual analytes in the sample, but these spectra can be assigned to a number of physiological or pathological conditions of the organism. In this way it is possible to create databases of typical spectra and compare them mutually. The proposed methods for identifying the properties of substances utilize and evaluate typical spectra shapes on the basis of which the sample can be accurately identified or studied.

Basic physicochemical methods used in the analysis of drugs, addictive drugs or food include chromatographic methods, i.e. a liquid or gas chromatography, electrophoresis or spectral methods, as a spectrophotometry, infrared or Raman spectroscopy. The extraction of analytes is one of the critical steps in the analytical determination of substance properties. As extraction agents, both polar and non-polar solvents are used. The polar solvents include most often acetonitrile, ethanol and methanol. One of the most important conditions for achieving high extraction efficiency is the choice of a suitable pH value. The pH value of respective extracting agent affects the solubility and/or ionization of analytes. The next step in the analysis may be to derivatize the sample. Derivatization reactions lead to the formation of detectable derivatives, to an increase in sensitivity and selectivity, an increase in the resolution, allowing the separation process itself, respectively. Generally, spectroscopic methods have less demands on the sample preparation compared to chromatographic methods. For example, Raman spectroscopy is one of the methods of vibration molecular spectroscopy that is used to chemically identify analytes contained in a sample. The method uses the so-called Raman effect or Raman scattering, which occurs when the laser beam interacts with the electrons of the sample under investigation. Since each substance exhibits a characteristic wavelength shift, this phenomenon can be used to non-destructively identify the chemical composition of a sample by creating a set of spectra typical for given analytes. The disadvantage of Raman scattering is its relatively low intensity, especially when compared to absorption or emission of radiation. Considerable purity of the analyzed sample is therefore necessary. Any fluorescence impurity or a possible photochemical reaction makes it impossible to carry out the analysis.

Another important method using a set of typical spectra represents also a mass spectrometry. This method can be used either alone or in combination with a separation technique.

Separate use of this method to determine the properties of substances without prior separation of samples can be problematic since the components of the laser ionized mixture and the degree of ionization are related to a number of factors. One of them is for example a type of matrix which is added to the sample as a part of the analysis. It absorbs most of the energy of the ionizing laser and transmits it to the sample. Because the operator does not know the composition of the sample, an incorrectly selected matrix may result in poor or partial ionization of the sample, resulting in distorting or disabling the analysis.

A few methods of analysis of substances which comprise comparing signals of a sample prior to and after UV irradiation have been known. They include methods disclosed in U.S. Patent Application having a pub. no. US 2013/0165330 A1 (D1) and U.S. Patent Application having a pub. no. US 2007/0141726 A1 (D2).

US 2013/0165330 A1 (D1) relates to a method of probing multiple targets in a biological sample comprising: (a) providing a biological sample containing multiple targets;(b) binding at least one probe, capable of binding to one or more targets present in the biological sample, to the biological sample and wherein said probe comprises at least one of a optical signal generator or a fluorescent signal generator; (c) observing a signal from the at least one probe bound in step (b); (d) contacting the biological sample comprising the bound probe of step (b) with an electron transfer reagent, wherein the electron transfer reagent is a borate salt, (e) irradiating the biological sample of step (d); (f) binding at least one probe to one or more targets present in the sample of step (e); and (g) observing a signal from the probe bound in step (f).

US 2007/0141726 A1 (D2) relates to a method for determining an analyte via interaction of the analyte with a: luminescent article, comprising: providing a sample suspected of containing an analyte; exposing the sample to a luminescent article comprising an outer layer and, if the analyte is present, allowing the analyte to become immobilized with respect to the article via interaction between the analyte and the outer layer, wherein the outer layer is modified by said interaction; determining a first emission of the luminescent article; exposing the nanoparticle to electromagnetic radiation for a period of time and under conditions sufficient to cause a change in a luminescence characteristic of the nanoparticle; determining a second emission of the luminescent article; and determining a variance between the first emission and the second emission indicative of the presence of the analyte,wherein modification of the outer layer increases the susceptibility of the article to a change in the luminescence characteristic upon exposure of the article to the electromagnetic radiation for the period of time and under the conditions, such that, in the absence of the analyte, exposure of the luminescent article to the electromagnetic radiation for the period of time and under the conditions does not result in said variance between the first and second emissions.

Neither of the methods of D1 an D2 involves the direct comparison of spectral data of untreated and irradiated samples and require the use of certain mediators (called "probes" or "luminiscent articles"). In order to obtain requisite data, both of these prior art methods require complicated procedures of modyfing the sample prior to and/or after the irradiation.

A method of reducing disulfide bonds of the protein to generate free thiol moieties with the help of UV light is known from US 6,313,274 B1 (D3).

US 6,313,274 B1 (D3) relates to a method of preparing a conjugate of a protein having one or more disulfide bonds, and a partner chemical which is reactive with free thiol moieties, which comprises subjecting the protein to ultraviolet radiation of an intensity and duration sufficient to reduce at least some of the disulfide bonds of the protein to generate free thiol moieties, without causing substantial aggregation of the protein, and then reacting the resulting photoactivated protein with said partner chemical.

### Summary of the Invention

The subject matter of the present invention relates to a method for identifying the properties of a sample, in particular a liquid sample, as defined in appended independent claim 1. Particular embodiments of methods in accordance with the present invention are defined in the appended dependent claims.

Preferably, the metal ions are selected from the group of Zn²⁺, Cu²⁺, Fe²⁺, Mg²⁺, Se²⁺ or Cd²⁺ and the disulfide bonds or the thiol group are selected from the group of cysteine, homocysteine, glutathione oxidized, glutathione reduced, S-Adenosyl-L-homocysteine, S-Adenosyl-methionine, coenzyme A, cystamine, acetylcysteine, gamma-L-Glutamyl-L-cysteine.

According to the invention, the sample is subjected to UV radiation at a wavelength of 250 to 400 nm.

The spectral changes induced by UV irradiation can be manifested by a change in the absorption or emission or excitation spectrum or as an increase or decrease in the absorption, emission or excitation signal. These changes can be mainly related to the formation of quantum nanocrystals, or to the change in the formation of radicals, or to the degradation of multiple chemical bonds, or to the change in photo-bleaching, or a combination of these changes.

The present invention provides a new, exceptional and unique fluorescence spectroscopic method which, based on typical spectra, can identify or distinguish individual samples or their properties.

Very advantageous are low demands on the sample preparation. For some samples, the sample should only be at least partially dissolved in distilled water or another solvent, for example dimethylsulfoxide, or mixed with a suitable buffer solution. The method has no special demands on the quality of the samples, thus it is universal and can be used in the industry, agriculture, food, pharmacy and research workplaces.

The speed of sample analysis represents an extraordinary advantage. The entire procedure, including the sample preparation, analysis and evaluation, can be performed within minutes. For example, the drug analysis does not exceed 10 min.

The method does not require any special treatment of analytical instruments and is compatible with instruments using ultraviolet and visible radiation for its operation.

The simplicity of analysis allows the use of case or hand-held analyzers, allowing analysis to be performed wherever it is needed.

### Brief Description of the Drawings

The results of exemplary embodiments and the method for identifying the properties of substances according to embodiments of the invention are shown in the accompanying drawings, in which:

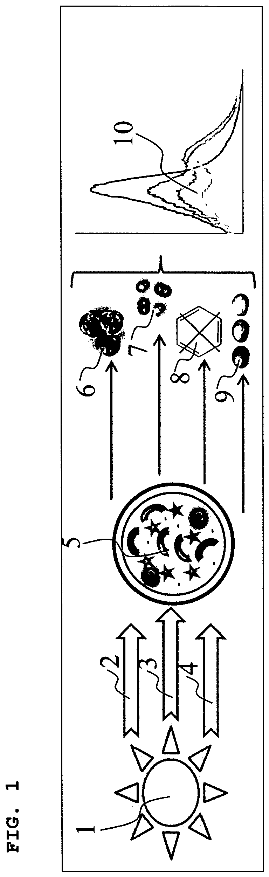

FIG. 1 schematically illustrates the nature of the method according to the invention, wherein the reference numeral 1 represents the ultraviolet radiation source, which is defined by the duration of irradiation as labeled by the reference numeral 2, by the wavelength as labeled by the reference numeral 3 and its intensity acting on the studied liquid sample as labeled by the reference numeral 4, and wherein the reference numeral 5 represents the sample being investigated where spectral changes are induced which are caused by the formation of fluorescent nanocrystals according to the reference numeral 6, the formation of free radicals shown by the reference numeral 7, degradation of chemical bonds shown by the reference numeral 8, and photo-bleaching illustrated by the reference numeral 9, wherein the changes can be recorded as absorption, fluorescence and excitation spectra, as shown by the reference numeral 10.

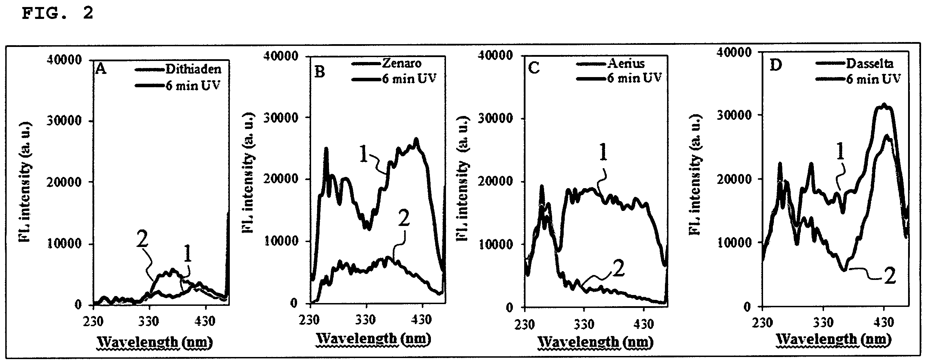

FIG. 2 shows excitation spectra in the range λₑₓ = 230-470 nm and λₑₘ = 500 nm of selected pharmacological agents, in this case antihistamines, dissolved in distilled water (20 mg / mL): the sample A is Dithiaden^{®} - manufactured by Zentiva, the sample B is Zenaro^{®} - manufactured by Zentiva, the sample C is Aerius^{®} - manufactured by SP Labo N. The sample D is Dasselta^{®} - manufactured by Krka d.d,. The curve 1 represents the excitation spectrum of the sample after 6 minutes of irradiation in the UV transilluminator (λₑₘ = 254 nm), the curve 2 represents the excitation spectrum of the sample in the native state, i.e. without UV irradiation. The data were acquired using the fluorescence analyzer Infinite^{®} M200 pro. The samples were irradiated and analyzed in 100 µL batches in a UV transparent 96 well plate.

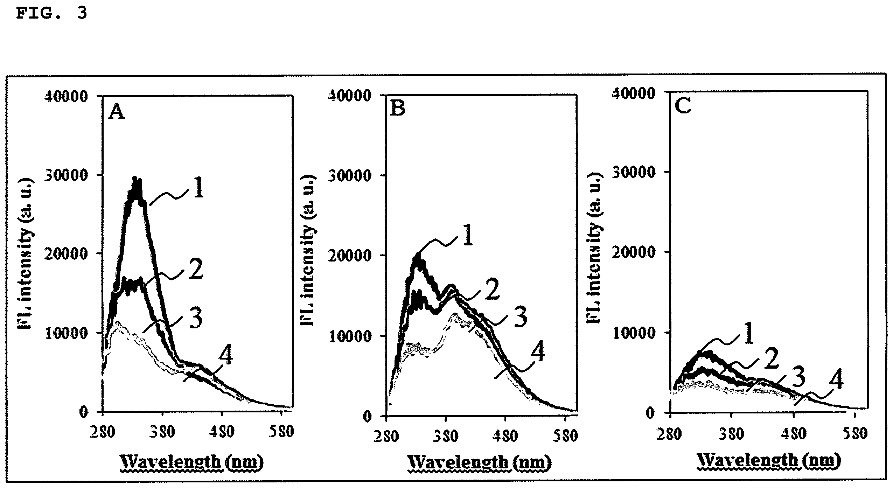

FIG. 3 shows emission spectra (λₑₓ = 250 nm and λₑₘ = 280 - 600 nm) of selected juices, where the sample labeled A represents orange juice from the company BASIC, denoted by the manufacturer as a 100% concentrate, the sample labeled B represents grapefruit juice from the same company and the sample labeled C represents apple juice, also from the same company. The curve 1 represents the result where 50 µL of the sample were mixed with 50 µL of 0.1 mol / liter phosphate buffer pH 7, the curve 2 represents the result where 50 µL of the sample diluted 1:1 with distilled water were mixed with 50 µL 0.1 mol / liter of phosphate buffer pH 7; the curve 3 represents the result where = 50 µL of a sample diluted 1 : 2 with distilled water were mixed with 50 µL of 0.1 mol / liter phosphate buffer pH 7 and the curve 4 represents the result where 50 µL of a sample diluted 1 : 4 with distilled water were mixed with 50 µL 0.1 mol / liter phosphate buffer pH 7. The samples were irradiated with a UV transilluminator (λₑₘ = 254 nm) in 100 µL batches in a UV transparent 96 well plate for 10 minutes. The data were acquired using the fluorescence analyzer Infinite^{®} M200 pro.

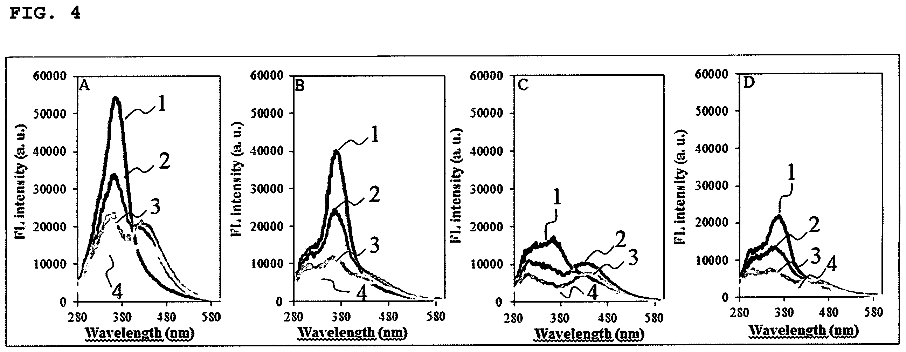

FIG. 4 shows the emission spectra (λₑₓ = 250 nm and λₑₘ = 280 - 600 nm) of selected varietal wines, wherein the sample A represents Riesling, the sample B represents Riesling Vla 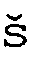 ský, the sample C represents red Traminer wine and the sample D represents Sauvignon. The curve 1 shows the result where 50 µL of the sample were mixed with 50 µL of 0.1 mol / liter phosphate buffer pH 7, the curve 2 shows the result where 50 µL of the sample diluted 1: 1 with distilled water were mixed with 50 µL 0.1 mol / liter phosphate buffer pH 7, the curve 3 shows the result where 50 µL of the sample diluted 1 : 2 with distilled water were mixed with 50 µL 0.1 mol / liter phosphate buffer pH 7 and the curve 4 shows the result where 50 µL of the sample diluted 1:4 with distilled water were mixed with 50 µL 0.1 mol / liter phosphate buffer pH 7. The samples were irradiated with the UV transilluminator (λₑₘ = 254 nm) for 100 µL in the UV transparent 96-well plate for 15 minutes. The data were taken with the Infinite^{®} M200 pro fluorescence analyzer.

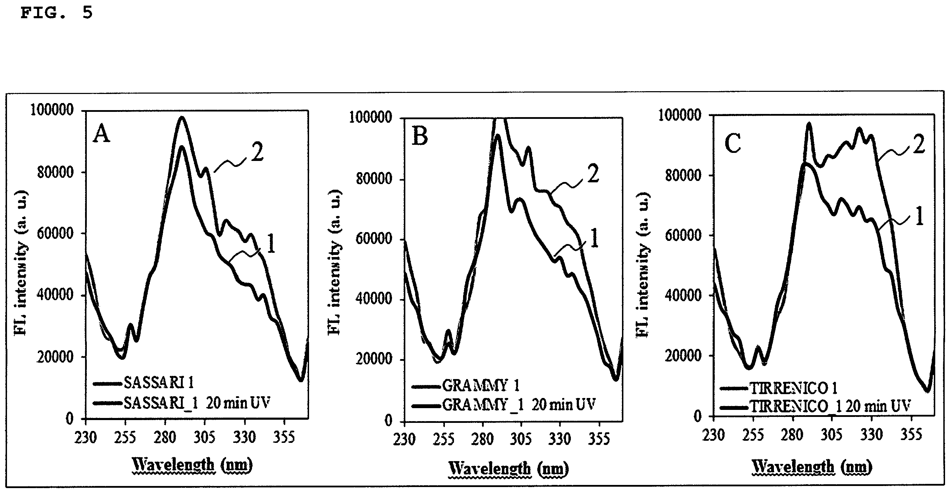

FIG 5 shows the excitation spectra in the range λₑₓ 230 - 370 nm and λₑₘ = 400 nm of selected tomato variety extracts, where the sample A represents the variety Sassari, the sample B represents the variety Grammy and the sample C represents the variety Tirrenico. The curve 1 shows the excitation spectrum of the sample after 20 minutes of irradiation in the UV transilluinator (λₑₘ = 254 nm); the curve 2 shows the excitation spectrum of the sample in its native state, i.e. not irradiated with UV radiation. The data were acquired with the Infinite^{®} M200 pro fluorescence analyzer. Samples were irradiated and analyzed in 100 µL batches in the UV transparent 96 well plate. The extracts, i.e. tomato pieces, were pressed and filtered through a 25 mm Syringe filter with a 0.2 µm membrane.

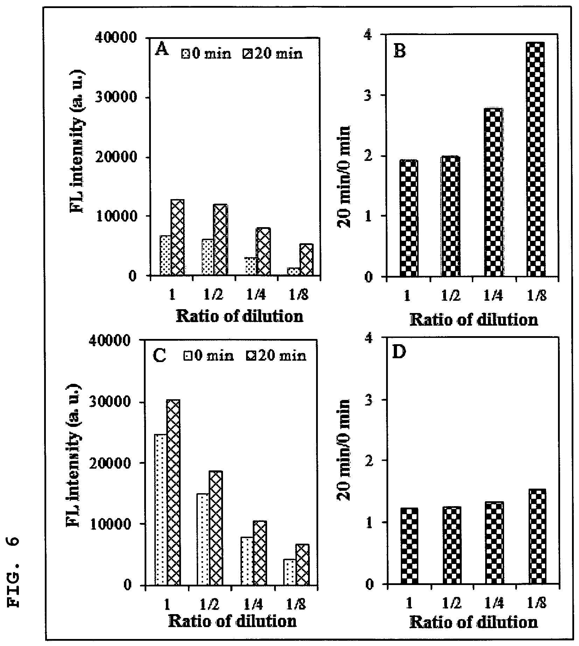

FIG. 6 shows a comparison of a urine sample of a healthy human and a patient with a diagnosed prostate cancer. The urine sample was diluted each with 1 : 1, 1 : 2, 1 : 4 and 1 : 8 0.1 M phosphate buffer pH 7 having the concentration 0, mol / liter and pipetted into the 100 µL UV transparent 96 well plate. The fluorescence intensity (λₑₓ - 400 nm and λₑₘ = 470 nm) of samples without UV irradiation and after 20 minutes irradiation with the UV transilluminator (λₑₘ = 254 nm) were then recorded. Graphs labeled A represent the fluorescence intensity of the urine control sample of the healthy human before irradiation (dotted bars) and after the UV exposure (cross-hatched bars). Graphs labeled B represent the fluorescence intensity (diced bars) of the control sample after and before the UV exposure, showing a significantly increasing trend. Graphs labeled C represent the fluorescence intensity of the urine sample of a prostate cancer patient prior to the irradiation (dotted bars) and after the UV exposure (cross-hatched bars), and graphs labeled D represent the proportion of fluorescence intensity of the urine sample of the prostate cancer patient after and before the UV exposure, wherein a noticeable growing trend is apparent. The data were taken with the Infinite^{®} M200 pro fluorescence analyzer.

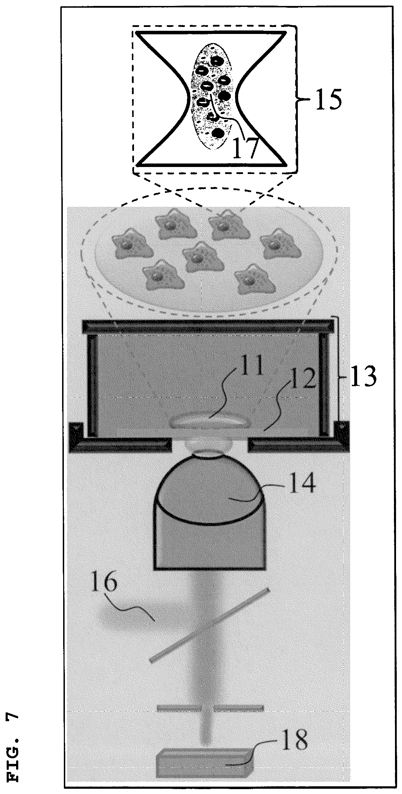

FIG. 7 shows a schematic performance of the intracellular analysis using a fluorescence correlation spectroscopy and a fluorescence microscope, wherein the reference numeral 11 represents a 10 µL sample that is dropped onto a slide 12, the reference numeral 13 represents a sample that is overlaid by a shielding sheath to remove the ambient illumination, the reference numeral 14 represents a fluorescent microscope objective focused on a confocal volume (typically 0.5 fL) into a selected portion of the sample under study 15, the reference numeral 16 represents an excitation laser (λₑₘ = 385 nm) which during the analysis induces fluorescent quantum dots in the sample, which are shown schematically under the reference numeral 17, and the reference numeral 18 represents a detector.

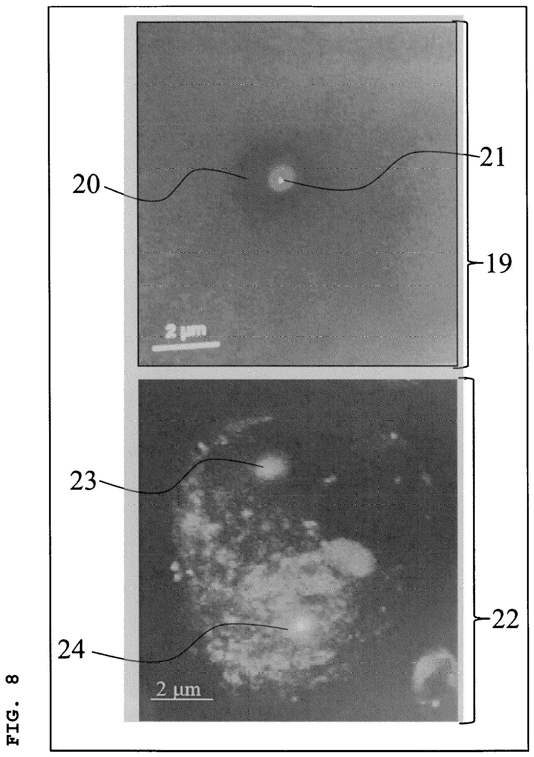

FIG. 8 under the reference numeral 19 shows a real photo of a 2% agarose gel which comprised zinc and cysteine recorded by the fluorescent microscope, wherein bleaching or loss of autofluorescence took place, as shown by the reference numeral 20, and the formation of fluorescent nanocrystals was occurred, as shown by the reference numeral 21. The reference numeral 22 shows a real photo of a single cell taken with a fluorescence microscope where fluorescent nanocrystals were formed, which are indicated by the reference numerals 23 and 24, whose spectral analysis provides information about a given location in the cell. Fluorescent nanocrystals labeled by the reference numerals 21, 23 and 24 were created during the analysis by means of the excitation laser having λ = 385 nm.

### Detailed description of examples and preferred embodiments of the invention

The invention will now be described in more detail by way of examples, which are, however, not intended to limit the scope of the claims. The method for identifying the properties of a sample comprising a substance as defined in claim 1 and according to the invention is schematically shown in Figure 1.

In the first step, a liquid sample is analyzed in its native state, i.e., not irradiated by UV radiation. The sample is then exposed to UV light at a precisely defined intensity for a few seconds or minutes. The irradiation time depends on the UV radiation intensity, wavelength and sample volume. UV radiation causes chemical changes in the sample, which transform as spectral changes specific to the relevant composition. UV photons have a high energy and can therefore break down chemical bonds. For example, chlorine does not react with alkanes under normal conditions. After the UV irradiation it begins to react because UV rays split the chemical bond in the Cl₂ molecule, which breaks down into extremely reactive radicals, which then react even with inert alkanes. The presence of the metal ions of the substance according to the invention, typically Zn²⁺, Cu²⁺, Fe²⁺, Mg²⁺, Se²⁺, Cd²⁺, and compounds having the disulfide bond (-SS-) and/or thiole group (-SH) of the substance according to the invention, especially cysteine, homocysteine, glutathione, S-Adenosyl-L-homocysteine, S-Adenosyl-methionine, coenzyme A, cystamine, acetylcysteine, gamma-L-Glutamyl-L-cysteine in the sample after interaction with UV can cause the formation of a number of nano and micro particles, complexes or clusters, which significantly influence the spectral properties of the studied matrix. The concentration of these precursors, i.e. metal ions and -SH, -S-S- containing compounds, required for cluster formation, is in the range of 1 nM to 3 mM. The analysis of the fluorescence spectral signals of such created clusters provides valuable information on the physical, chemical or biological properties of the sample. Therefore, changes in absorption and fluorescence, i.e. the excitation or emission signals, their shape, waveform, intensity, the lifetime of fluorescence, the fluorescent anisotropy, photobleaching, fluorophore diffusion coefficients before and after UV irradiation are evaluated. The content of thiol groups or disulfide substances to be investigated within the scope of the invention is not added to the sample. Each substance, with only a few exceptions, contains them in trace amounts which are absolutely sufficient for the conventional analytical methods of the invention to achieve technical effects. These are substances that are naturally contained in each sample.

If a sample is changed by adding another component, by substitution for another component, or changing the type and concentration of thiol compounds (i.e. -SH, -S-S-containing compounds) or metal ions, the described method captures this change. Similarly, the method captures natural differences in samples, such as plant varieties or disease-related clinical changes.

The intended methodological procedures can be implemented into a conventional desktop spectrometer or even in the form of a handheld device, allowing on-site analyzes to be performed.

As a source of UV radiation, a table transilluminator (λₑₘ = 254 nm) or a UV laser beam (λₑₘ = 385 nm) can typically be used. Other light sources of suitable wavelengths such as electroluminescent diodes, LEDs and lamps can also be used. The changes induced by the UV emitter are typical of the chemical composition of the sample and can be monitored by conventional spectroscopic instrumentations, i.e. by means fluorescence spectroscopy, correlation fluorescence spectroscopy and the like.

### Example 1 (not forming part of the invention but representing background art that is useful for understanding the invention)

The test substance in this case is a chemical preparation, particularly a drug. This substance is first homogenized to a fine fraction by grinding and weighed to 10-20 mg doses. 1 mL of distilled water or another solvent such as dimethylsulfoxide is added to the drug. The sample is vigorously stirred or shaken for one minute and after dissolution, at least partial, 50 µL of the resulting suspension is mixed with 50 µL of 0.1 mol / liter phosphate buffer pH 7 and / or distilled water in a UV transparent microtiter plate or spectroscopic cuvette. The sample is first analyzed in its native state using spectroscopic methods of excitation absorption and / or radiation emission. An example can be an absorption scan in the range of 230 to 800 nm or an excitation scan at λₑₓ = 230 to 470 nm and λₑₘ = 500 nm (optionally 450 nm). Afterwards, the sample plate is exposed to UV radiation (λₑₘ = 254 nm) in a trans illuminator or other radiation source for 6 minutes. Then the spectral analysis is repeated under the same parameters. The results of the analysis are unique spectra that are typical of a given analyte or sample composition. The spectra obtained are created based on the UV-induced degradation of the test substance, i.e. by photobleaching or dissociation of functional groups, which leads to the formation of free radicals and subsequent typical reactions.

This procedure and its results correspond to FIG. 2. The analysis of other liquid, dissolved or suspension samples is carried out in an analogous way. In this way, the profiling of chemical mixtures can be performed to identify them.

### Example 2

The test substance in this case is a fruit drink or wine. The beverage is diluted to four concentrations by the half-dilution with distilled water. The beverage is then mixed 1 : 1 with 0.1 mol / liter phosphate buffer pH 7 in a microtiter plate or spectroscopic cuvette. The plate is exposed for 10 to 15 minutes to UV radiation (λₑₘ = 254 nm) in a transilluminator or by means of other radiation source. Emission or excitation spectra are then recorded. An example can be an emission scan at λₑₓ = 250 nm and λₑₘ = 280 to 800 nm. The emission or excitation spectra recorded are typical of the beverage. The spectra obtained are the result of UV radiation caused by the photooxidation of thiol groups (SH) and subsequent interaction with metal ions. Furthermore, free radicals are generated under the influence of UV radiation, which interact with other molecules.

This procedure and its results correspond to FIGs. 3 and 4. In this way, it is possible to determine precisely what fruit the juice originates from, or whether the necessary technology has been observed in processing, storage, peeling prior to pressing, or similar beverage preparation operations.

### Example 3

The subject under examination corresponds to vegetable or fruit extracts. The extract or juice obtained by pressing or leaching from fruit or vegetables is filtered to remove the pulp or other impurities and solid fractions. Then the sample is mixed 1 . 1 with 0.1 mol / liter phosphate buffer pH 7 in a microtiter plate or spectroscopic cuvette. The sample is first analyzed in its native state, i.e. without UV irradiation by spectroscopic methods, i.e. the absorption, excitation and emission of radiation. An example can be an excitation scan at λₑₓ = 230 to 370 nm and λₑₘ = 400 nm. Afterwards, the sample plate is exposed to UV radiation (λₘ = 254 nm) for 20 minutes in a trans illuminator or other UV device. Then the spectral analysis is repeated under the same parameters. The results of the analysis are unique spectra, which are typical of a given variety of fruits or vegetables. The spectra obtained are the result of UV radiation caused by the photooxidation of thiol groups (SH) and subsequent interaction with metal ions. Furthermore, free radicals are generated under the influence of UV radiation, which interact with other molecules.

This procedure and its results correspond to FIG. 5. In this way, different varieties of vegetables and fruits can be distinguished and identified.

### Example 4

The subject under investigation corresponds to clinical specimens. As in the previous examples, biological or clinical specimens such as blood plasma or urine may be used. These samples can be used to study the physiological or pathological conditions of an organism associated with metal ions and -S-S-, -SH molecules. A change in the concentration of metal ions and / or -SS-, -SH compounds in the study sample results in a change in the emission or excitation spectrum of the shape or as an increase or decrease in the emission or excitation signal at a particular wavelength (for example λₑₓ = 400 nm and λₑₘ = 470 nm) after UV irradiation.

This procedure and its results correspond to FIG. 6. In this way, control tests can be performed to detect serious diseases at an early stage.

### Example 5 (not forming part of the invention but representing background art that is useful for understanding the invention)

In this case, the investigation focuses on the localization and identification of cellular processes. A cell line or histological section is placed or dropped in the volume 1-50 µL on a glass slide and placed in a fluorescent microscope. Using the microscope and appropriate software, cell locations to be analyzed are selected. Next, a laser with a suitable wavelength (typically λₑₘ = 385 nm) is selected, which for a few seconds or minutes, most often in the range of 1 to 90 seconds, induces cluster formation at the analysis site. These clusters are evaluated in real time or after the analysis. The resulting clusters are typical of a given site in a cell, for its composition and for the biological or chemical processes taking place therein. An example of the analysis performance is shown in FIG. 7.

### Industrial applicability

The use of the method according to the invention can be expected in areas where it is necessary to monitor the composition of pharmacological agents, the quality and composition of beverages or foods, or to diagnose serious diseases from body fluids or at the level of a histological section or cell samples.

The method makes it possible, for example, to detect counterfeiting or to determine illegal additives in mixtures such as medicines, food, and many other types of substances. Similarly, the method can be used to detect a number of misdemeanors associated with food counterfeiting, or failure to adhere to a given technology or recipe in beverage and food production.

Furthermore, the procedure may also serve to identify a drug by the rescue service during the transfer of a patient to the hospital. The procedure provides a cheap and fast screening tool to hospitals or research institutes by the study of clinical specimens. Using advanced spectroscopic techniques, such as fluorescence correlation spectroscopy, the method can localize or monitor cellular processes within a single cell. The intended method is compatible with all spectroscopic methods using electromagnetic waves in the classical range.

### Legend for Figures of Drawings

### Fig. 1

1) The source of ultraviolet radiation that can be characterized according to:
2) duration time,
3) wavelength, and
4) intensity.

### Exposure of a sample

5) by ultraviolet radiation causes spectral changes based on:
6) formation of fluorescence nanoparticles/complexes,
7) formation of free radicals,
8) degradation of chemical bonds, and/or
9) photobleaching.

These changes are typical for sample and can by monitored as a 10) absorbance, fluorescence and/or excitation spectra.

### Fig. 7

11) The drop of sample containing prokaryotic and/or eukaryotic cells is placed on
12) plain glass microscope slide and
13) covered with a shield box to remove a ambient light.
14) The lens is focused on selected part of cell (sample).

Into the
15) confocal volume is concentrated
16) the excitation laser and causing
17) the formation of fluorescent nanocrystals/complexes, which are detected by
18) the detector.

### Fig. 8

19) Real image from fluorescence microscopy of 2% agarose gel containing zinc and cysteine. The point of analysis is characterized by
20) photobleaching (loss of autofluorescence) and by formation of new
21) fluorescent nanocrystals.
22) Real photo of single cell from a fluorescence microscope. The site of analysis in the cell is characterized by the formation of
23) and
24) fluorescent nanocrystals. Spectral analysis of these fluorescent nanocrystals provides information about a given cell site.

## Claims

1. A method for identifying the properties of a sample, in particular a liquid sample, **characterized in that** a substance in the sample consists of on the one hand metal ions and on the other hand a thiol group or disulphide bonds, and that the sample is subjected to a sequence of steps consisting of:
(i) first, either subjecting said sample to fluorescence spectral analysis in the original or initial untreated state, i.e. not irradiated by UV radiation, the result of the fluorescence spectral analysis is a unique fluorescence spectrum of the sample in the original or initial untreated state, or providing from an information database a unique fluorescence spectrum of the sample in the original or initial untreated state, i.e. not irradiated by UV radiation,
(ii) then, subjecting the sample in the original or initial untreated state to UV radiation at a wavelength selected from the range from 250 to 400 nm, thereby causing spectral changes in the fluorescence spectrum of the sample that are characteristic of the substance in the sample, and thereby providing a sample in the treated state, i.e. irradiated by UV radiation,
(iii) then, subjecting said sample in the treated state to fluorescence spectral analysis under the same conditions as in step (i), the result of the fluorescence spectral analysis is a unique fluorescence spectrum of the sample in the treated state, and
(iv) then, identifying properties of the sample based on the spectral changes that are characteristic of the substance in the sample derived from the differences between the unique fluorescence spectra of steps (i) and (iii).

2. The method for identifying the properties of a sample, in particular a liquid sample, according to claim 1, **characterized in that** the metal ions are selected from the group of of Zn²⁺, Cu²⁺, Fe²⁺, Mg²⁺, Se²⁺ or Cd²⁺.

3. The method for identifying the properties of a sample, in particular a liquid sample, according to claim 1, **characterized in that** the thiol group is from the group of cysteine, homocysteine, glutathione oxidized, glutathione reduced, S-adenosyl-L-homocysteine, S-adenosylmethionine, coenzyme A, cystamine, acetylcysteine, gamma-L-glutamyl-L-cysteine.

4. The method for identifying the properties of a sample, in particular a liquid sample, according to claim 1, **characterized in that** the UV radiation generates quantum nanocrystals or free radicals in the sample, degrades chemical bonds in the sample, or causes photo-bleaching in the sample.

5. The method for identifying the properties of a sample, in particular liquid sample, according to claim 1, **characterized in that** a concentration change of the metal ions, in particular Zn²⁺, Cu²⁺, Fe²⁺, Mg²⁺, Se²⁺ or Cd²⁺ and the thiol group, in particular cysteine, homocysteine, glutathione oxidized, glutathione reduced, S-Adenosyl-L-homocysteine, S-Adenosyl-methionine, coenzyme A, cystamine, acetylcysteine, gamma-L-glutamyl-L-cysteine in the sample results in a spectral change in the fluorescence spectrum of the sample related to the absorption or emission or excitation spectrum, or to an increase or decrease of the absorption, emission or excitation signal.

6. The method for identifying the properties of a sample, in particular a liquid sample, according to claim 1, **characterized in that** a concentration change of the metal ions and the thiol group results in a spectral change in the fluorescence spectrum of the sample related to the formation of quantum nanocrystals.

7. The method for identifying the properties of a sample, in particular a liquid sample, according to claim 1, **characterized in that** a concentration change of the metal ions and the thiol group results in a spectral change in the fluorescence spectrum of the sample related to the formation of radicals.

8. The method for identifying the properties of a sample, in particular a liquid sample, according to claim 1, **characterized in that** a concentration change of the metal ions and the thiol group results in a spectral change in the fluorescence spectrum of the sample related to the degradation of multiple chemical bonds.

9. The method for identifying the properties of a sample, in particular a liquid sample, according to claim 1, **characterized in that** a concentration change of the metal ions and the thiol group results in a spectral change in the fluorescence spectrum of the sample related to photo-bleaching.

## Patentansprüche

1. Verfahren zur Identifizierung der Eigenschaften einer Probe, insbesondere einer flüssigen Probe, **dadurch gekennzeichnet, dass** eine Substanz in der Probe einerseits aus Metallionen und andererseits aus einer Thiolgruppe oder Disulfidbindungen besteht, und dass die Probe einer Folge von Schritten unterzogen wird, die darin besteht, dass:
(i) Zunächst wird die Probe entweder einer Fluoreszenzspektralanalyse im ursprünglichen oder anfänglichen unbehandelten, d.h. nicht mit UV-Strahlung bestrahlten Zustand unterzogen, wobei das Ergebnis der Fluoreszenzspektralanalyse ein eindeutiges Fluoreszenzspektrum der Probe im ursprünglichen oder anfänglichen unbehandelten Zustand ist, oder es wird aus einer Informationsdatenbank ein eindeutiges Fluoreszenzspektrum der Probe im ursprünglichen oder anfänglichen unbehandelten, d.h. nicht mit UV-Strahlung bestrahlten Zustand bereitgestellt,
(ii) anschließend wird die Probe im ursprünglichen oder anfänglichen unbehandelten Zustand einer UV-Strahlung mit einer Wellenlänge aus dem Bereich von 250 bis 400 nm ausgesetzt, wodurch spektrale Veränderungen im Fluoreszenzspektrum der Probe verursacht werden, die für die Substanz in der Probe charakteristisch sind, und dadurch eine Probe im behandelten Zustand, d.h. mit UV-Strahlung bestrahlt, bereitgestellt wird,
(iii) anschließend Unterziehen der Probe im behandelten Zustand einer Fluoreszenzspektralanalyse unter den gleichen Bedingungen wie in Schritt (i), wobei das Ergebnis der Fluoreszenzspektralanalyse ein eindeutiges Fluoreszenzspektrum der Probe im behandelten Zustand ist, und
(iv) anschließend Identifizierung von Eigenschaften der Probe anhand der spektralen Veränderungen, die für die Substanz in der Probe charakteristisch sind, abgeleitet aus den Unterschieden zwischen den eindeutigen Fluoreszenzspektren der Schritte (i) und (iii).

2. Verfahren zur Identifizierung der Eigenschaften einer Probe, insbesondere einer flüssigen Probe, nach Anspruch 1, **dadurch gekennzeichnet, dass** die Metallionen aus der Gruppe Zn2+, Cu2+, Fe2+, Mg2+, Se2+ oder Cd2+ ausgewählt sind.

3. Verfahren zur Identifizierung der Eigenschaften einer Probe, insbesondere einer flüssigen Probe, nach Anspruch 1, **dadurch gekennzeichnet, dass** die Thiolgruppe aus der Gruppe Cystein, Homocystein, Glutathion oxidiert, Glutathion reduziert, S-Adenosyl-L-Homocystein, S-Adenosylmethionin, Coenzym A, Cystamin, gamma-L-Glutamyl-L-Cystein stammt.

4. Verfahren zur Identifizierung der Eigenschaften einer Probe, insbesondere einer flüssigen Probe, nach Anspruch 1, **dadurch gekennzeichnet, dass** die UV-Strahlung Quanteneinkristalle aus freien Radikalen in der Probe erzeugt, chemische Bindungen in der Probe abbaut oder ein Ausbleichen der Probe durch Licht bewirkt.

5. Verfahren zur Identifizierung der Eigenschaften einer Probe, insbesondere einer flüssigen Probe, nach Anspruch 1, **dadurch gekennzeichnet, dass** eine Konzentrationsänderung der Metallionen, insbesondere Zn2+ , Cu2+ , Fe2+ , Mg2+, Se2+ oder Cd2+ und der Thiolgruppe, insbesondere Cystein, Homocystein, Glutathion oxidiert, Glutathion reduziert, S-Adenosyl-L-Homocystein, S-Adenosyl-Methionin, Coenzym A, Cystamin, Acetylcystein, gamma-L-Gluzamyl-L-Cystein in der Probe führt zu einer spektralen Veränderung des Fluoreszenzspektrums der Probe in Bezug auf das Absorptions- oder Emissions- oder Anregungsspektrum oder zu einer Zunahme oder Abnahme des Absorptions-, Emissions- oder Anregungssignals.

6. Verfahren zur Identifizierung der Eigenschaften einer Probe, insbesondere einer flüssigen Probe, nach Anspruch 1, **dadurch gekennzeichnet, dass** eine Konzentrationsänderung der Metallionen und der Thiolgruppe zu einer spektralen Änderung des Fluoreszenzspektrums der Probe führt, die mit der Bildung von Quanten-Nanokristallen zusammenhängt.

7. Verfahren zur Identifizierung der Eigenschaften einer Probe, insbesondere einer flüssigen Probe, nach Anspruch 1, **dadurch gekennzeichnet, dass** eine Konzentrationsänderung der Metallionen und der Thiolgruppe zu einer spektralen Änderung des Fluoreszenzspektrums der Probe führt, die mit der Bildung von Radikalen verbunden ist.

8. Verfahren zur Identifizierung der Eigenschaften einer Probe, insbesondere einer flüssigen Probe, nach Anspruch 1, **dadurch gekennzeichnet, dass** eine Konzentrationsänderung der Metallionen und der Thiolgruppe zu einer spektralen Änderung im Fluoreszenzspektrum der Probe führt, die mit dem Abbau von chemischen Mehrfachbindungen verbunden ist.

9. Verfahren zur Identifizierung der Eigenschaften einer Probe, insbesondere einer flüssigen Probe, nach Anspruch 1, **dadurch gekennzeichnet, dass** eine Konzentrationsänderung der Metallionen und der Thiolgruppe zu einer spektralen Änderung des Fluoreszenzspektrums der Probe führt, die mit dem Photobleichen verbunden ist.

## Revendications

1. Procédé d'identification des propriétés d'un échantillon, en particulier d'un échantillon liquide, **caractérisé en ce que** la substance dans l'échantillon est constituée d'ions métalliques d'une part et de groupes thiol ou de liaisons disulfure d'une autre part, et également **en ce que** l'échantillon est soumis à une séquence d'étapes qui comprennent:
(i) tout d'abord, ledit échantillon dans son état d'origine ou initial non modifié, c'est-à-dire sans traitement avec un rayonnement UV, est soumis à une analyse spectrale de fluorescence, le résultat de l'analyse spectrale de fluorescence étant un spectre de fluorescence unique de l'échantillon dans son état d'origine ou initial non modifié, c'est-à-dire sans traitement avec un rayonnement UV,
(ii) puis l'échantillon dans son état d'origine ou initial non modifié est soumis à un traitement avec un rayonnement UV d'une longueur d'onde choisie dans la gamme de 250 à 400 nm, provoquant de telles modifications spectrales dans le spectre de fluorescence de l'échantillon qui sont caractéristiques de la substance présente dans l'échantillon, obtenant l'échantillon à l'état modifié, c'est-à-dire dans l'état après traitement avec un rayonnement UV,
(iii) puis ledit échantillon à l'état modifié est soumis à une analyse spectrale de fluorescence dans les mêmes conditions qu'à l'étape (i), dans laquelle le résultat de l'analyse spectrale de fluorescence est le spectre de fluorescence unique de l'échantillon à l'état modifié, et
(iv) puis, les propriétés de l'échantillon sont identifiées sur la base des changements spectraux dérivés des différences entre les spectres de fluorescence uniques des étapes (i) et (iii) et caractéristiques de la substance présente dans l'échantillon.

2. Procédé d'identification des propriétés d'un échantillon, en particulier d'un échantillon liquide, selon la revendication 1, **caractérisé en ce que** les ions métalliques sont choisis dans le groupe de
Zn²⁺, Cu²⁺, Fe²⁺, Mg²⁺, Se²⁺ ou Cd²⁺.

3. Procédé d'identification des propriétés d'un échantillon, en particulier d'un échantillon liquide, selon la revendication 1, **caractérisé en ce que** le groupe thiol est choisi parmi le groupe cystéine, le groupe homocystéine, le groupe glutathion oxydé, le groupe glutathion réduit, le groupe S-adénosyl-L-homocystéine, le groupe S-adénosylméthionine, le groupe coenzyme A, le groupe cystamine, le groupe acétylcystéine, le groupe gamma-L-glutamyl-L-cystéine.

4. Procédé d'identification des propriétés d'un échantillon, en particulier d'un échantillon liquide, selon la revendication 1, **caractérisé en ce que** le rayonnement UV dans l'échantillon génère des nanocristaux quantiques ou des radicaux libres, dégrade les liaisons chimiques dans l'échantillon ou induit un photoblanchiment de l'échantillon.

5. Procédé d'identification des propriétés d'un échantillon, en particulier d'un échantillon liquide, selon la revendication 1, **caractérisé en ce qu'**une modification de la concentration des ions métalliques dans l'échantillon, en particulier de Zn²⁺, Cu²⁺, Fe²⁺, Mg²⁺, Se²⁺ ou Cd²⁺, et du groupe thiol dans l'échantillon, en particulier du groupe cystéine, groupe homocystéine, groupe glutathion oxydé, groupe glutathion réduit, groupe S-adénosyl-L-homocystéine, groupe S-adénosylméthionine, groupe coenzyme A, groupe cystamine, groupe acétylcystéine et du groupe gamma-L-glutamyl-L-cystéine, entraîne une modification spectrale du spectre de fluorescence liée à l'absorption, à l'émission ou à l'excitation du spectre, ou à une augmentation ou une diminution du signal d'absorption, d'émission ou d'excitation.

6. Procédé d'identification des propriétés d'un échantillon, en particulier d'un échantillon liquide, selon la revendication 1, **caractérisé en ce qu'**une modification de la concentration des ions métalliques et du groupe thiol entraîne une modification spectrale du spectre de fluorescence de l'échantillon, qui est liée à la formation de nanocristaux quantiques.

7. Procédé d'identification des propriétés d'un échantillon, en particulier d'un échantillon liquide, selon la revendication 1, **caractérisé en ce qu'**une modification de la concentration des ions métalliques et du groupe thiol entraîne une modification spectrale du spectre de fluorescence de l'échantillon, qui est liée à la formation de radicaux.

8. Procédé d'identification des propriétés d'un échantillon, en particulier d'un échantillon liquide, selon la revendication 1, **caractérisé en ce qu'**une modification de la concentration des ions métalliques et du groupe thiol entraîne une modification spectrale du spectre de fluorescence de l'échantillon, qui est liée à la dégradation de plusieurs liaisons chimiques.

9. Procédé d'identification des propriétés d'un échantillon, en particulier d'un échantillon liquide, selon la revendication 1, **caractérisé en ce qu'**une modification de la concentration des ions métalliques et du groupe thiol entraîne une modification spectrale du spectre de fluorescence de l'échantillon, qui est liée au photoblanchiment.
